# EUROPEAN PATENT SPECIFICATION

(11) **EP 2 967 844 B1**
(45) Date of publication and mention of the grant of the patent: **06.12.2017**
(21) Application number: 14764654.1
(22) Date of filing: 15.03.2014
(51) Int. Cl.: A61F 2/20

(54) **APPARATUS FOR TREATING GLOTTIC INSUFFICIENCY**
VORRICHTUNG ZUR BEHANDLUNG VON STIMMRITZENFEHLERN
APPAREILS POUR TRAITER L'INSUFFISANCE GLOTTIQUE

(30) Priority: 15.03.2013 US 201361790081 P; 16.09.2013 US 201361878045 P; 16.09.2013 US 201361878047 P
(43) Date of publication of application: 20.01.2016
(73) Proprietor: Aprevent Medical Inc., Grand Cayman KY1-1112 (KY); Ho, Guan-Min, 1050 Vienna (AT)
(72) Inventor: HO, Guan-Min, 1050 Vienna (AT); CHEN, Yenyu, 1050 Vienna (AT)
(74) Representative: Foster, Mark Charles
(86) International application number: PCT/US2014/030067
(87) International publication number: WO 2014/145327

(56) References cited:
- US-A- 5 897 579
- US-A1- 2004 254 642
- US-A1- 2006 037 617
- US-A1- 2006 161 045
- US-A1- 2008 066 766
- US-A1- 2008 071 230
- US-A1- 2010 023 125
- US-A1- 2011 144 558
- US-A1- 2011 301 580
- US-A1- 2012 150 293
- US-A1- 2012 150 293
- US-A1- 2012 296 243

## Description

### BACKGROUND

Dysphagia associated with aspiration pneumonia often occurs in patients with neurological disorders. The neurological disorders may be caused by stroke, brain surgery, head and/or spinal cord trauma, oropharyngeal disease, radiation therapy, cardiac/thoracic surgery, autoimmune or other degenerative neurologic diseases. And the aspiration pneumonia may be mainly caused by glottic insufficiency, due to vocal fold paralysis with/without swallowing dysfunction. Stroke patients with aspiration symptoms may have a seven-time higher risk in developing aspiration pneumonia than other types of patients. For these stroke patients, even after recovery, there is still a relatively high incidence of dysphagia associated with aspiration pneumonia.

Conventional surgical techniques to treat dysphagia and glottic insufficiency may include Type I Medialization Thyroplasty (MT) procedure and Arytenoid Adduction (AA) procedure. Type I MT procedure is the main phonosurgical procedure performed in patients with glottic insufficiency. The primary limitations of Type I MT procedure include the inability to close a wide posterior glottal chink and restore the physiological swallowing steps, like laryngeal elevation and vocal fold movement. For patients with vocal cord paralysis and a significant posterior glottic gap after the Type I MT procedure, an AA procedure may be performed subsequently to close the incompletely closed posterior glottis. Still, one limitation of the AA procedure, associated with the posterior airway closure, is an increased frequency of postoperative airway complaints after the AA procedure, due to post-operative tissue edema in the glottis area. Further, Type I MT and AA procedures may not be suitable for patients having difficulty with prolonged periods of supine positioning or intolerable for long lasting surgical procedures.

Since the above two procedures either use implants or suture fixation technique, a common complaint, resulting from these procedures, is the inability to precisely adjust the implant or sutures intra-operatively and the inability of post-operative implant- and suture-adjustments. Specifically, it is difficult to accurately perform intraoperative adjustment of implant due to edematous swelling of vocal tract mucosa caused by these procedures. For example, carving an implant during surgery may result in prolonged operation time and suboptimal shaping of the implant. Furthermore, these implants cannot be postoperatively adjusted at all.

US2011/144558 discloses an implant for treating medical disorders that includes a first chamber having a flexible outer layer that surrounds a flexible inner layer, a second chamber in communication with the first chamber, and a fluid transfer assembly adapted for transferring fluid between the second chamber and the first chamber for selectively modifying the rigidity of the first chamber. The implant includes at least one restraining element in contact with the flexible outer and inner layers for restricting volume expansion of the first chamber as the fluid is transferred into the first chamber. The first chamber is adapted to become more rigid as the fluid is transferred into the first chamber and more flexible as the fluid is removed from the first chamber. The first chamber is implantable within the soft tissue of a patient such as a tongue, soft palate, pharyngeal wall, urinary tract, rectum, trachea or stomach.

US2012/150293 discloses a laryngeal implant for treating glottic insufficiency, including a displacement member positionable in a larynx such that at least a portion of the displacement member is disposed between thyroid cartilage and a vocal fold of the larynx. The displacement member is at least one of selectively adjustable in volume and selectively adjustable in compressibility when the displacement member is positioned in the larynx to selectively position the vocal fold in a medial displacement position.

### SUMMARY

In one aspect, the present invention provides an implant system as defined in claim 1.

Optional features of the implant system are defined in the dependent claims.

### BRIEF DESCRIPTION OF THE DRAWINGS

Figure 1A illustrates various views of larynx anatomy.
Figure 1B illustrates various symptoms of glottic insufficiency and the medical procedures that can treat glottic insufficiency in order to reduce aspiration pneumonia, according to one or more embodiments of the present disclosure.
Figure 2 illustrates diagrams of a surgical device configured to perform one or more medical treatments, according to one or more embodiments of the present disclosure.
Figure 3 illustrates diagrams showing a surgical device configured to perform various medical treatments after a cartilage opening is created.
Figure 4 illustrates diagrams of a fixture device configured to be placed into a cartilage opening, according to one or more embodiments of the present disclosure.
Figure 5 illustrates diagrams of various implants configured to treat glottic insufficiency, according to one or more embodiments of the present disclosure.
Figure 6 illustrates various components that may be associated with an implant for the treatment of glottic insufficiency, according to one or more embodiments of the present disclosure.
Figure 7 illustrates various diagrams of a port configured to connect to an implant for the treatment of glottic insufficiency, according to one or more embodiments of the present disclosure.
Figure 8 illustrates diagrams of an implant system configured to treat glottic insufficiency, according to one or more embodiments of the present disclosure.
Figure 9 shows a flow diagram illustrating a process to treat a patient's glottic insufficiency, according to one or more embodiments of the present disclosure.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made. It will be readily understood that the aspects of the present disclosure, as generally described herein, and illustrated in the Figures, can be arranged, substituted, combined, and designed in a wide variety of different configurations, all of which are explicitly contemplated herein.

The present disclosure describes apparatuses and methods to treat dysphagia, glottic insufficiency (due to neuromuscular incoordination or disordered cooperation between the intrinsic muscles), and the poor closure of larynx opening (due to uncoordinated bending of epiglottis and delayed or absence of laryngeal elevation). The disclosed treatments may improve the glottic closure, reduce the incidence of aspiration, and thus preventing the aspiration's sequelae, such as aspiration pneumonia. Furthermore, the disclosed treatments may enable the postoperative adjustment of implants, and reduce the risk of postoperative airway compromise. As a result, the disclosed treatments may shorten the operation time during the surgical procedures, and effectively reduce/prevent aspiration pneumonia after the surgical procedures.

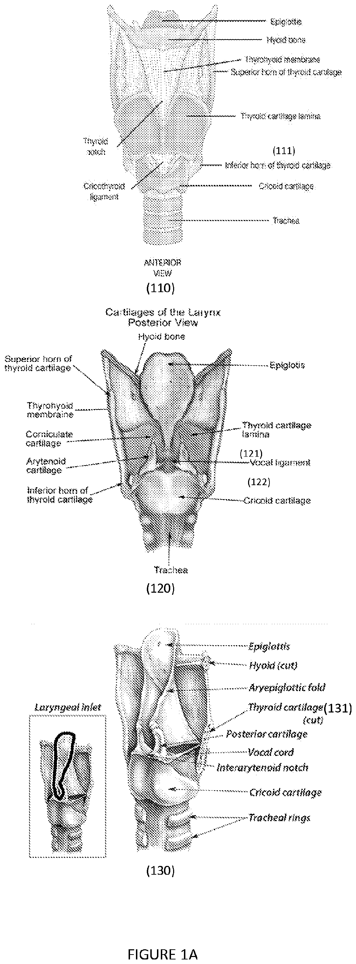

Figure 1A illustrates various views of larynx anatomy. In Figure 1A, drawing 110 may show an anterior view of a patient's larynx, drawing 120 may show a posterior view of the patient's larynx, and drawing 130 may show a lateral view of the laryngeal cartilages.

Drawing 110 may show the anatomy of a patient's larynx in an anterior view, with the outer side of the thyroid cartilage 111 exposed. Drawing 120 may show the same patient's larynx in a posterior view, with the inner side of the thyroid cartilage 121 exposed. Drawing 120 may further show the patient's vocal ligament 122, which are enclosed within the patient's vocal folds (not shown in the drawing 120). Drawing 130 may show the same patient's laryngeal cartilages (including the thyroid cartilage 131) in an angled lateral view. In one example, the space surrounded by the laryngeal cartilages, as well as the tissues and organs connected to these cartilages, may be deemed a "paraglottic space." In other words, the paraglottic space may be a space bounded by the thyroid cartilage and the surrounding various membranes. In some examples, the laryngeal box may include "paraglottic space" and "pre-epiglottic space," which is a space anterior to the epiglottis and laterally extending into the paraglottic space.

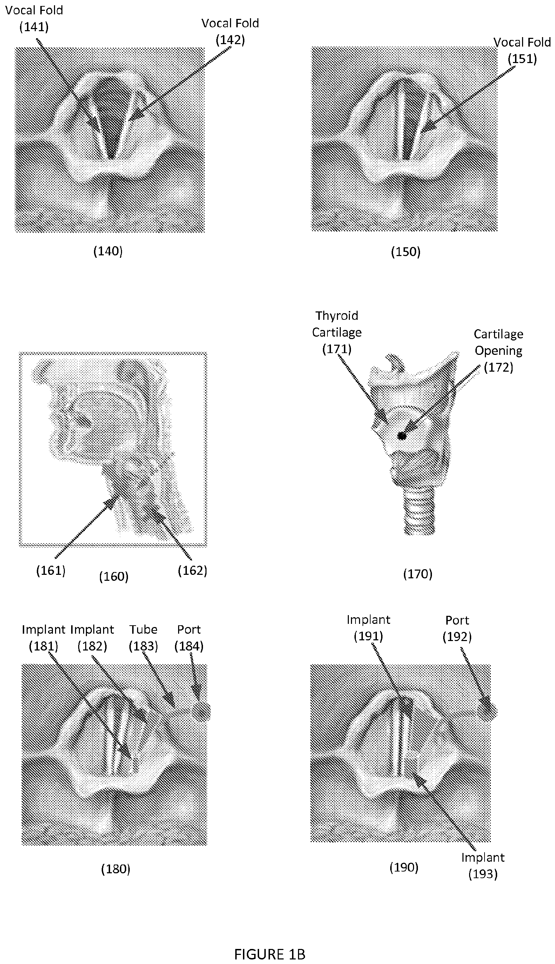

Figure 1B illustrates various symptoms of glottic insufficiency and the medical procedures that can treat glottic insufficiency, in order to reduce aspiration pneumonia, according to one or more examples. In Figure 1B, drawing 140 may show a healthy vocal cord, drawing 150 may show a left side paralyzed vocal cord with glottic insufficiency, and drawing 160 may illustrate a sagittal view of a patient's larynx when the patient is having incoordination between epiglottis bending movements, laryngeal elevation, and vocal closure. The surgical treatments presented in the present disclosure may be illustrated in drawings 170, 180, and 190, accordingly.

Drawing 140 illustrates an example healthy vocal cord, having two lateralized vocal folds 141 and 142. During normal breathing, swallowing or speaking, the two vocal folds 141 and 142 may open and close in unison. Before a person swallows food, the food or drink may first be crushed and/or mixed into a pasty mass known as a bolus. During swallowing, the person's extrinsic and intrinsic muscles may cooperate to prevent food or drink from entering the glottis. For example, the person's extrinsic muscles may elevate the larynx and bend the epiglottis over the entrance to the glottis, so that the bolus can glide across the epiglottis rather than falling into the larynx. While this movement is under way, the person's intrinsic muscles may close the glottis. Should any food particles or liquids touch the surface of the vestibular or vocal folds 141 and 142, a cough reflex may be triggered to prevent the material from entering the glottis.

Drawing 150 illustrates an example unhealthy vocal cord, having a paralyzed vocal fold 151 unable to move to a fully lateralized/medialized position. In other words, the paralyzed vocal fold 151 cannot be opened and closed in unison with the other vocal fold, leaving an opening or gap in the glottis. Thus, during swallowing, the bolus may inadvertently slip into the larynx and subsequently into the trachea, bronchus and lungs, which may lead to infection and pneumonia. Further, as shown in drawing 160, for some of the stroke patients, due to impaired neurological stimulus, the trigger of epiglottis bending, the vocal closure 161, and/or the laryngeal elevation 162 may be delayed or absent, leading to incoordination of their extrinsic and intrinsic muscle movements. As a result, the patient may have difficulty swallowing and choking as well.

In one example, as shown in the drawing 170, to treat a patient's glottic insufficiency and/or swallowing difficulty, a surgeon or a medical machinery may utilize a surgical device to create a cartilage opening 172 in the patient's thyroid cartilage 171. The surgical device may perform certain functions such as drilling, shaping, space expansion (e.g., "dissection"), and instrument/implant delivery. Afterwards, the surgeon or the medical machinery may place an implant system through the cartilage opening 172 into the paraglottic space behind the patient's thyroid cartilage 171. The implant system may be configured to facilitate epiglottis bending or perform laryngeal elevation so that a paralyzed larynx may be better closed during bolus swallowing.

In one example, as shown in drawing 180, the implant system may contain an implant 181, an implant 182, one or more tubes 183, and a port 184. The implant 181 may be deployed to the space between the aryepiglottic muscle and the thyroid cartilage, the so called "paraglottic space", to position a paralyzed vocal fold to a nearly complete medialized position. Further, the implant 181 may extend its position vertically, thus reaching the supraglottic space, resulting in elevation of larynx and acceleration of epiglottis closure during swallowing. The implant 182 may be extended into the para-epiglottic space, to enhance closure of a paralyzed vocal fold to a nearly complete or fully medialized position.

Drawing 190 may show the implant system's therapeutic effects after an implant 191 (corresponding to the implant 182 of the drawing 180) in the implant system is planted into the paraglottic space. The shape and size of the implant 191 may be adjustable by adding/removing filler to/from the implant 191 via the port 192 during operation (intra-operatively). After operation (postoperatively), when a complication occurs, the implant 191 may be readjusted to handle airway compromise. Thus, the implant 191 may effectively facilitate the paralyzed vocal fold to reach the medialized position. As a result, the risk of aspiration pneumonia can be greatly reduced. The implant 193, which corresponds to the implant 181 of the drawing 180, may be similarly adjusted and readjusted either intra-operatively or postoperatively. Thus, the implant system may allow faster operation time, which in turn would increase patients' tolerance to accept this procedure.

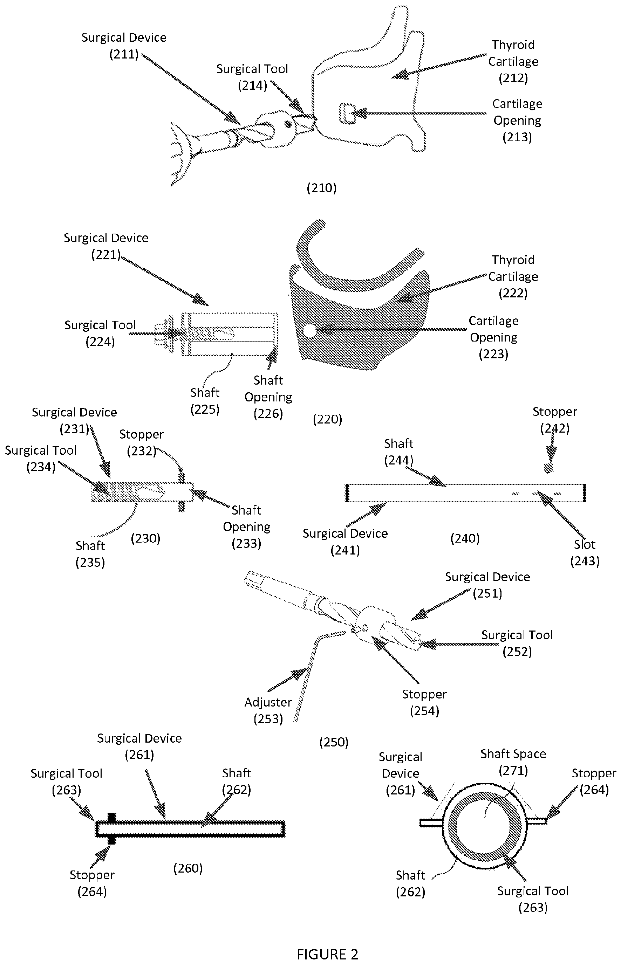

Figure 2 illustrates diagrams of a surgical device configured to perform one or more medical treatments, according to one or more examples. In Figure 2, drawings 210 and 220 may show a surgical device being used on a patient's thyroid cartilage. Drawings 230, 240, 250, 260, and 270 may illustrate the details of the surgical device as well as its components therein.

In one example, a surgeon or a medical machinery may use a surgical device 211 to operate on the patient's thyroid cartilage 212. Specifically, the surgeon may manually operate the surgical device 211. Or the surgical device 211 may be attached to the medical machinery, which may be a machine that can be configured or programmed to operate the surgical device 211. Specifically, the surgeon or the medical machinery may first use the surgical device 211 to create a cartilage opening 213 in the patient's thyroid cartilage 212. Afterward, the surgeon or the medical machinery may utilize the same surgical device 211 to make one or more space expansions, and deliver one or more implants, into the patient's paraglottic space. In one example, the surgical device 211 may include a surgical tool 214, which may be, without limitations, a cutter, a drill, saw, or punch (e.g., a Kerrison punch). Further, the surgical tool 214 may be manually, electrically, mechanically, or magnetically driven.

During operation, under local anesthesia, the surgeon or the medical machinery may first make a 5-6cm incision through the patient's platysma. The surgeon may then elevate the patient's superior and inferior flaps to expose the thyroid cartilage 212. Afterward, the surgeon or the medical machinery may use the surgical tool 214 to remove a chunk of the thyroid cartilage 212 and create the cartilage opening 213. The tissues and organs originally underneath the portion of the thyroid cartilage 212 being removed may then be exposed through the cartilage opening 213. The cartilage opening 213 may have a circular shape when the surgical tool 214 is a drill or a circular saw. The cartilage opening 213 may also have a substantially rectangular shape when the surgical tool 214 is a saw or a punch. In some examples, the location of the cartilage opening 213 may be created anywhere on the thyroid cartilage 212. Afterward, the surgeon or the medical machinery may preserve the inner perichondrium and elevate the inner perichondrium off the thyroid cartilage, in order to insert an implant through the cartilage opening.

In one example, the surgical device 211 may be shown in detail in the drawing 220. Specifically, the surgical device 221, the thyroid cartilage 222, the cartilage opening 223, and the surgical tool 224 correspond to their respective counterparts in the drawing 210. Further, the surgical device 221 may optionally include one or more shafts 225, each of which may have an inner space ("lumen") and a shaft opening 226 at one end. In one example, the inner space may be a channel or a tunnel, allowing a surgical tool 224, an introducing instrument, an instrument for dissection, a fiber optical instruction, or an implant to pass through. For example, the surgeon or the medical machinery may be configured to guide a guide wire through the lumen of the shaft 225 toward the shaft opening 226.

In one example, the surgeon or the medical machinery may aim the shaft opening 226 toward the cartilage opening 223, in order to guide a medical instrument through the cartilage opening 223 and into the inner space of the thyroid cartilage 222. In another example, the surgical device 221 may have multiple shafts 225. In this case, the surgeon or the medical machinery may place the surgical tool 224 through one shaft 225 in order to create the cartilage opening 223, and utilize another shaft 225 to place the medical instrument through the cartilage opening 223. Alternatively, a single shaft 225 may have multiple shaft openings located on one end and/or a lateral side of the shaft 225, allowing multiple medical instruments to go through the multiple shaft openings via the same lumen of the shaft 225.

In one example, the surgical device 221 may be shown in further detail in the drawing 230. Specifically, the surgical device 231, the surgical tool 234, the shaft 235, and the shaft opening 233 correspond to their respective counterparts in the drawing 220. In drawing 230's example, the surgical tool 234 may be placed inside of the lumen of the shaft 235. During operation, the surgical tool 234 may be extended out of the shafting opening 233 to reach the thyroid cartilage and create the cartilage opening as shown above. The shaft 235 may further contain one or more stoppers 232 located inside or on the outer surface of the surgical device 231. The stoppers 232 may be placed near the shaft opening 233, in order to stop the surgical tool 234 from overcutting or extending too deep into the thyroid cartilage. The distance between the stopper 232 and the shaft opening 233 may be referred to as the "cutting depth." In other words, the surgeon or the medical machinery may control how deep the surgical tool 234 can cut into the thyroid cartilage by adjusting the cutting depth. For example, the cutting depth may be configured to be substantially the same as the thickness of the thyroid cartilage. In this case, once the surgical tool 234 cuts through the thyroid cartilage, the stopper 232 may prevent the surgical tool 234 from cutting further into the tissues and organs underneath the thyroid cartilage.

In one example, the surgical device 231 may be shown in further details in the drawing 240. Specifically, a surgical device 241 may have a shaft 244 having one or more fixed slots 243. Each of the fixed slots 243 may be a side opening having a fixed location on the lateral side of the shaft 244, and may be configured to hold a stopper 242. Thus, the surgical device 241 may be configured with multiple slots 243 to adjust its surgical tool's cutting depth. Once a specific stopper 242 is placed into a specific slot 243, the surgical device 241 may have a specific cutting depth, and may be configured to stop the advancing of the surgical tool beyond the specific stopper 242. The stopper 242 may be removed from the specific slot 243 and placed into another slot 243. In this case, the surgical device 241 may then be configured to have a different cutting depth.

In one example, the drawing 250 may show, in part, a surgical device 251 similar to the surgical device 211 of the drawing 210. Specifically, the surgical device 251 may have a surgical tool 252 and a stopper 254. The stopper 254 may be attached to the outside surface of the surgical tool 252, and may be tightened to the surgical tool 252 using an adjuster 253 (e.g., a screw driver or a fastener). In other words, the adjuster 253 may tighten a screw on the stopper 254 to prevent the stopper 254 from moving on the surgical tool 252. Thus, the stopper 254 may allow the cutting depth of the surgical device 251 to be adjusted continuously between zero length and the full length of the surgical tool 252. During operation, once the stopper 254 is in contact with the outer surface of the thyroid cartilage, it would prevent the surgical tool 252 from advancing further into the thyroid cartilage.

In one example, the drawing 260 may show, in part, a lateral-view of a surgical device 261 having a surgical tool 263 and a shaft 262 integrated into one piece. In other words, the shaft opening of the shaft 262 may be the surgical tool 263, which may have saw-like teeth that can be used to cut through any contacting material. And the surgical tool 263 may have a hollow inner space acting as the lumen of the shaft 262. During operation, the surgeon or the medical machinery may place the surgical tool 263 in contact with the thyroid cartilage, and rotate the shaft 262 either manually or using a power source. The rotating of the shaft 262 may allow the surgical tool 263 to drive into the thyroid cartilage to create a cartilage opening. In the meantime, another medical instrument may be placed into the lumen of the shaft 262 either when the surgical tool 263 is in operation, or after the surgical tool 263 completed its operation.

In one example, the drawing 270 may show, in part, a cross-sectional frontal view of the surgical device 261 of the drawing 260. In other words, the surgical device 261 in the drawing 270 may have a hollow shaft space 271. Further, the surgical tool 263 may be attached to the shaft 262. During operation, a medical instrument may be guided through the hollow shaft space of the shaft 262, either during or after the surgical tool 263 creating a cartilage opening.

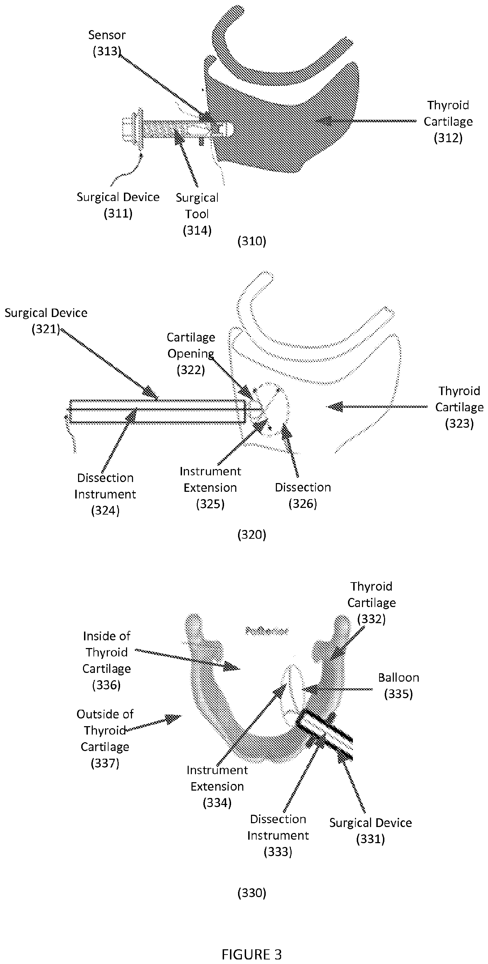

Figure 3 illustrates diagrams showing a surgical device configured to perform various medical treatments after a cartilage opening is created. In one example, the drawing 310 illustrates a surgical device 311 being used to create a cartilage opening in a thyroid cartilage 312. The surgical device 311 may be configured with a sensor 313 either attached to the surgical device 311's surgical tool 314 or its shaft. During operation, the sensor 313 may detect various measurements of the surgical tool 314 in order to prevent over-cutting. For example, when the surgical tool 314 is a drill, the sensor 313 may be a torque sensor configured to detect the amount of torque applied to the thyroid cartilage by the drill. Once the torque sensor senses that the torque is either below a particular and configurable torque threshold or disappeared, the surgical device 311 may determine that the thyroid cartilage 312 is drilled through, and slow down or stop the drill. In another example, the sensor 313 may be a pressure sensor to detect an amount of force applied by the surgical tool 314 and/or the surgical device 311's shaft to the thyroid cartilage 312. Upon a determination that the amount of force is below a particular and configurable force threshold or reaching zero, the surgical device 311 may determine that the thyroid cartilage 312 is drilled through, and stop the surgical tool 314 accordingly.

In one example, the drawing 320 may illustrate a surgical device 321 utilized to create a dissection 326 after a surgical tool of the surgical device 321 created a cartilage opening 322 in the thyroid cartilage 323. During operation, the surgeon or the medical machinery may insert a dissection instrument 324 through the shaft and the shaft lumen of the surgical device 321, into the paraglottic space behind the thyroid cartilage 323 via the cartilage opening 322. Afterward, the surgeon or the medical machinery may apply forces to the dissection instrument 324, in order to stretch, cut, or remove the tissues around the cartilage opening 322 and create the dissection 326. The surgeon or the medical machinery may then use the dissection 326 to facilitate the deployment of an implant into the patient's paraglottic space.

In one example, the dissection instrument 324 may be an angled device (e.g., a hook) to expand or separate the tissues around the cartilage opening 322 in order to create a small dissection 326. The dissection instrument 324 may also be a guide wire having one or more instrument extensions 325 (e.g., branches) at the guide wire's one end. The surgeon or the medical machinery may direct the guide wire into the cartilage opening 322, and create the dissection 326 by applying forces to the guide wire. The applied forces may then be transmitted to the instrument extensions 325, which may in turn push, pull, or separate the tissues around the cartilage opening 322. Further, the surgeon or the medical machinery may put a balloon onto the instrument extensions 325 of the guide wire, and use the guide wire to direct the balloon into the cartilage opening 322. The surgeon or the medical machinery may preload the balloon onto the instrument extensions 325, or load the balloon through a specific shaft of the surgical device 321 after its surgical tool created the cartilage opening 322. The tips of the dissection extensions 325 may be blunt, so that the forces applied to the instrument extensions 325 may not penetrate the balloon. Afterward, the surgeon or the medical machinery may control the guide wire to expand the balloon, in order to create a desired dissection 326 using the balloon.

In one example, the drawing 330 may show a horizontal view of the paraglottic space surrounded by a patient's thyroid cartilage 332 during the creation of a dissection using a surgical device 331. The drawing 330 may show, in part, the surgical device 331 and a dissection instrument 333 passing through a shaft of the surgical device 331. Further, the dissection instrument 333 may contain one or more instrument extensions 334, and a balloon 335 is placed on the tips of these instrument extensions 334. Thus, the surgeon or the medical machinery may first control the dissection instrument 333 to guide the balloon 335 into the cartilage opening, and then apply forces to the dissection instrument 333 to direct the balloon 335 into a desired location in the paraglottic space. Alternatively, the surgeon or the medical machinery may fill the balloon 335 with air or liquid, in order to expand the balloon 335 and create the dissection. For clarification purposes, a space that is surrounded by the thyroid cartilage 332 may be referred to be "inside of the thyroid cartilage 336", and a space that is on the opposite side of the thyroid cartilage 332 with respect to the "inside of thyroid cartilage" may be referred to as being "outside of the thyroid cartilage."

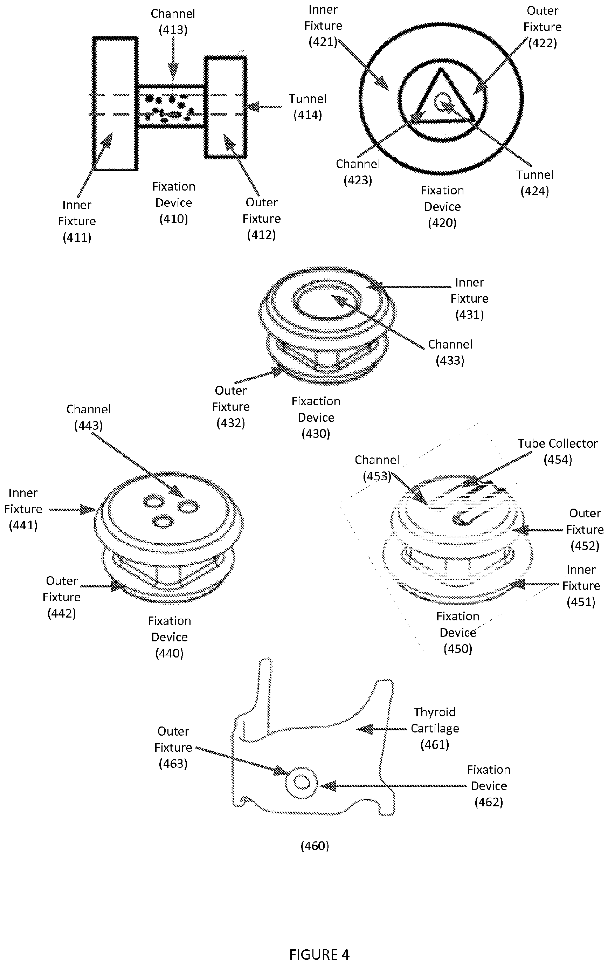

Figure 4 illustrates diagrams of a fixture device, configured to be placed into a cartilage opening, according to one or more embodiments of the present disclosure. In Figure 4, drawing 410 may show a lateral view of a fixation device 410. Drawing 420 may show a frontal-view of a fixation device 420. Drawing 430 may show a top view of a fixation device 430 having one channel. Drawing 440 may show a top view of a fixation device 440 having multiple channels. Drawing 450 may show a bottom view of a fixation device 450 having multiple channels. And drawing 460 may show a fixation device 462 being placed into a cartilage opening.

In one embodiment, the fixation device 410 may contain an inner fixture 411, an outer fixture 412, and one or more channels 413. Once the fixation device 410 is placed into a cartilage opening in a thyroid cartilage, the inner fixture 411 may be in contact with an inner surface of the thyroid cartilage that is located beneath the thyroid cartilage, and the outer fixture 412 may be in contact with an outer surface of the thyroid cartilage that is located outside of the thyroid cartilage. The terms "outer" and "inner" may be relative to each other, as the fixation device 410 may be placed into the cartilage opening in such a way that the inner fixture 411 may actually be located outside of the thyroid cartilage, and the outer fixture 412 may be located inside of the thyroid cartilage. In other words, the inner fixture 411 may be positioned on one side of the thyroid cartilage, and the outer fixture 412 may be positioned on the other side of the thyroid cartilage.

In one embodiment, the inner fixture 411 or the outer fixture 412 may have a diameter size that is larger than the diameter size of the cartilage opening. The length of the portion ("waist") of the channel 413 between the inner fixture 411 and the outer fixture 412 may be substantially the same as a thickness of the thyroid cartilage at around the cartilage opening. Thus, the inner fixture 411 and the outer fixture 412 may clamp the fixation device 410 to the thyroid cartilage, so that the fixation device 410 may be securely placed into the cartilage opening without moving around or slipping out of the cartilage opening.

In one embodiment, the fixation device 410 may include a channel 413 positioned between the inner fixture 411 and the outer fixture 412. The channel 413 may have a tunnel 414 that passes through the inner fixture 411, the body of the channel 413, and the outer fixture 412. Specifically, the tunnel 414 may be the hollow space starting from a first tunnel opening on the outer fixture 412, all the way through the inner body of the channel 413, and exiting at a second tunnel opening on the inner fixture 411. In other words, the channel 413 may be construed as the materials surrounding the tunnel 414. When the fixation device 410 is placed in the cartilage opening, the tunnel 414 may form an opening that allows access to the space inside of the thyroid cartilage. Thus, the fixation device 410 may be configured to provide a medical instrument or an implant an entry to pass through the cartilage opening and enter into the inside of the thyroid cartilage, and/or to hold and stabilize a part of an implant or a tube that couples to the implant plated inside of the thyroid cartilage.

In one embodiment, the channel 413 or the waist of the fixation device 410 may have a rough outer side surface. As a result, when the fixation device 410 is placed into the cartilage opening, the rough outer surface of the channel 413, which may be in contact with the wall of the cartilage opening, may generate sufficient frictions to stabilize the fixation device 410 from slipping or rotating in the cartilage opening. Further, the cross-section view of the channel 413 may have a non-circular outer shape (e.g., triangular or rectangular) as well, in order to further reduce the slippage or rotations of the fixation device 410 in the cartilage opening.

In one embodiment, the fixation device 420 may show a frontal view of the fixation device 410. Specifically, the fixation device 420 may have an inner fixture 421 corresponding to the inner fixture 411, an outer fixture 422 corresponding to the outer fixture 412, a channel 423 corresponding to the channel 413, and a tunnel 424 corresponding to the tunnel 414 of the fixation device 410. In one embodiment, as illustrated by the fixation device 420, the inner fixture 421 may have a larger cross-section diameter than the outer fixture 422's. Alternatively, the inner fixture 421's cross-section diameter may substantially equal, or smaller than the outer fixture 422's cross-section diameter. The cross-section view of the channel 423 may be non-circular (e.g., triangular in Figure 4). Once the fixation device 420 is placed in the cartilage opening, the tunnel 424 may allow access through the cartilage opening, or be configured to hold a tube that couples to an implant placed in the paraglottic space.

In one embodiment, the fixation device 430 may show a top view of the fixation device 410. Specifically, the fixation device 430 may have an inner fixture 431 corresponding to the inner fixture 411, an outer fixture 432 corresponding to the outer fixture 412, a channel 433 corresponding to the channel 413 of the fixation device 410. Please note that the channel 433 may be referring to the materials that surround a tunnel, not the tunnel itself. For example, a cross-section view of the channel 433 may show a substantially rectangular outer shape and a substantially circular inner shape.

In one embodiment, the fixation device 440 may have an inner fixture 441, and outer fixture 442, and one or more channels 443. Specifically, the fixation device 440 may have one or more channels 443 each of which surrounds a corresponding tunnel that passes through the fixation device 440. Furthermore, each channel 443 may be configured to hold a tube that enters the paraglottic space, or allow a medical instrument to pass, through the cartilage opening. Please note that the three channels 443 contained in the fixation device 440 shown in Figure 4 are for illustrative purposes. The fixation device 440 may contain multiple channels 443 forming a combined structure with a cross-section outer shape that is substantially rectangular.

In one embodiment, the fixation device 450 may show a top view of the fixation device 440. In other words, the fixation device 450 may have an inner fixture 451 corresponding to the inner fixture 441, an outer fixture 452 corresponding to the outer fixture 442, and one or more channels 453 corresponding to the one or more channels 443 of the fixation device 440. Further, on the outer surface of the outer fixture 452, there are one or more tube collectors 454 associated with the channels 453. Each tube collector 454 may refer to the materials that form a carved-out shape on the surface of the outer fixture 452. Each of the tube collectors 454 may also be connected with one of the channels 453, and may be configured to hold a flexible tube that passes through a particular channel 453's channel opening. In other words, once a tube passes through a channel 453, it may be bended and pushed into a tube collector 454 that is collected with the channel 453. Once the tube is placed into the tube collector 454, it may be securely fastened to the fixation device 450. Further, a secured tube won't easily be pushed/pulled in/out of the fixation device 450. The tube collector 454 may also be configured to guide the laying of the tube toward a specific orientation/direction.

In one embodiment, the drawing 460 may show a fixation device 462 being placed into/through a cartilage opening of the thyroid cartilage 461. The fixation device 462 may be made using materials such as elastomer, silicone block, polymer, synthetic material, ENT synthetic, porous polyethylene, ENT synthetic-PIFE, silicon elastomer, polyethylene, and polyurethane. As illustrated in Figure 4, the fixation device 462 may be flexible, so that it can restore back to its original shape after being squeezed into the cartilage opening. After insertion, the fixation device 462 may have an outer fixture 463 exposed to the outside of the thyroid cartilage 461. The inner fixture of the fixation device 462 is located inside of the thyroid cartilage 461, and is not shown in Figure 4. In one embodiment, a surgeon or a medical machinery may also secure (e.g., by stitching) the fixation device 462 on a surface/side of the thyroid cartilage 461 or on any tissues around the thyroid cartilage 461. Furthermore, the fixation device 462 may be placed at or near an edge of the thyroid cartilage 461 by having the fixation device 462's inner fixture and outer fixture clamping on a part of the thyroid cartilage 461. Even when the fixation device 460 is not placed in/through the cartilage opening, it can still be used to guide a medical instrument (e.g., a guide wire), an implant, or a tube into the paraglottic space via the fixation device 462's channel.

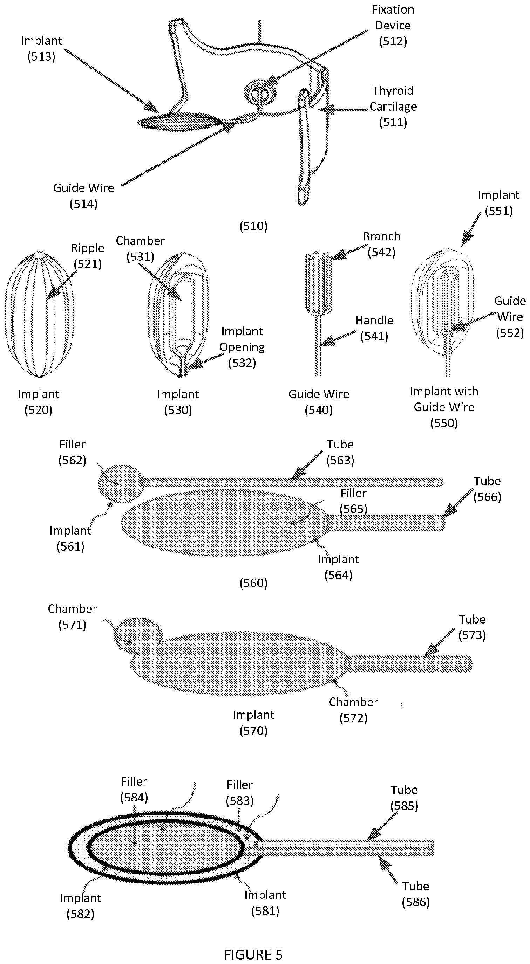

Figure 5 illustrates diagrams of various implants configured to treat glottic insufficiency, according to one or more embodiments of the present disclosure. In Figure 5, a drawing 510 may show an implant being placed into a patient's paraglottic space. The drawings 520, 530, 540, and 550 may show various views of an implant. The drawings 560, 570, and 580 may illustrate how the implants may be utilized during operation.

In one embodiment, a fixation device 512 may be placed into a cartilage opening of the patient's thyroid cartilage 511, after a dissection is created in the patient's paraglottic space. A surgeon or a medical machinery may then deploy an implant 513 into the patient's paraglottic space using a guide wire 514. Specifically, the surgeon or the medical machinery may attach/insert one end of the guide wire 514 to/into the implant 513, and control the other end of the guide wire 514 to move the implant 513 into a desired location in the patient's paraglottic space.

In one embodiment, the implant 513 may be further illustrated by the implants 520 and 530. The implant 520 may be made of materials that are flexible and stretchable. For example, the implant 520 may be made of materials such as silastic, hydroxyapatite, titanium, Gore-Tex, and autologous bone implants. Further, the implant 520 may be inflatable/expandable or deflatable/contractible based on an amount of filler in the implant 520. Specifically, when the implant 520 is filled with a filler, the implant 520 may be inflated or expanded in volume or size. Likewise, when the filler is extracted or removed from the implant 520, the implant 520 may be deflated or shrunk in volume or size. For example, the implant 520 may be a balloon. Alternatively, the implant 520 may be made of bio-compatible materials (e.g., silicon) or bio-degradable materials.

In one embodiment, the implant 520 may have a smooth or an uneven outer surface. For example, the implant 520 may have nipple-like materials (e.g., apophysis) on its outer surface, or have multiple stripes of ripples 521 that extend from one end to the other end of the implant 520. These added materials on the outer surface of the implant 520 may provide frictions. When the implant 520 is placed inside a patient's body, the frictions between the uneven surface of the implant 520 and the neighboring tissues may prevent the implant 520 from drifting around inside the patient's body. The uneven outer surface may also help minimizes the pressure from the implant 520 to the neighboring tissues, thereby lowering the possibility of pressure-induced ischemia.

In one embodiment, the filler for filling the implant 520 may be some type of fluids, such as water, gas, saline, Calcium Hydroxyapatite (CaHA), or hyaluronic acid. The CaHA and hyaluronic acid filler may be moldable, and may be adjusted into a shape that conforms to a specific dissection in the paraglottic space. Further, the CaHA and hyaluronic acid may be thermally treated to remold. And a filler material such as Hyaluronic acid may be dissipated via enzymatic pathways. In one embodiment, in order to stabilize the shape of an inflated implant 520, the filler materials may include particles with diameters that are larger than 65 micrometers (µm). In other words, the particles having larger-than 65 µm diameters may be suitable to maintain or restore the shape of the implant 520, even after external pressure is applied to the implant 520.

In one embodiment, the implant 530 may show a cross-section view of its internal. Specifically, the implant 530 may have one or more inner chambers 531, as well as one or more implant openings 532 located at one of its end. Each inner chamber 531 may be fillable with filler or be left empty. The inner chamber 531 may also be expandable or non-expandable. Further, the implant opening 532 may be used for adding or extracting filler from the implant 530 or the inner chamber 531. Alternatively, a guide wire 540 may be inserted into the implant 530 via the implant opening 532. In one embodiment, the guide wire 540 may have a handle 541 and one or more branches 542 located at one end of the handle 541. The tip of the branches 542 may be blunt, in order to provide frictions or prevent puncturing the implant.

In one embodiment, the implant with guide wire 550 may show a guide wire 552 inserted into an implant 551 via its implant opening. Specifically, the guide wire 552 may be inserted into one of the inner chambers of the implant 551. When the specific inner chamber the guide wire 552 is inserted into is non-expandable and made of rigid material, the force applied to the implant 551 via the guide wire 552 may be shielded by the specific inner chamber. Afterward, a surgeon or a medical machinery may manipulate the handle 541 to guide the implant 551 into a desired location in a patient's paraglottic space.

In one embodiment, the drawing 560 may show implants with various shapes and sizes. Specifically, the implant 561, which is attached with a tube 563, may have a ball shape. In other words, the implant 561 may have a homogeneous flexibility in all directions. When filler 562 is filled into or extracted from the implant 561 via the tube 563, the implant 561 may expand or shrink evenly in all directions. In comparison, the implant 564, which is attached with a tube 566, may have an inhomogeneous flexibility. In other words, when filler 565 is introduced into or removed from the implant 564 via the tube 566, the implant 564 may inflate or deflate unevenly, or asymmetrically. For example, the implant may expand or shrink more in horizontal directions than in vertical directions. In another embodiment, the inner chambers of the implant 564 may also have homogeneous and/or inhomogeneous flexibility. And the surgeon or the medical machinery may utilize the homogeneous and inhomogeneous flexibility of the implant 564 or the inner chambers contained therein to selectively apply forces to the tissues next to the implant 564.

In one embodiment, the drawing 570 may show an implant 570 having one or more independent inner chambers 571 and 572, and one or more tubes 573 for connecting to the one or more inner chambers 571 and 572. Specifically, each inner chambers 571 and 572 may be connected with one corresponding tubes 573. Thus, a surgeon or a medical machinery may utilize a corresponding tube 573 to control the inner chamber 571, and utilize another corresponding tube 573 to control the inner chamber 572.

In one embodiment, the tube 573 may contain multiple tube channels, each of which may be independently controlled for adjustment of the space limited by membranes wrapped around the tube 573. Specifically, the inner chamber 571 may be constructed by wrapping a membrane around the tube 573, and may be connected to one or more openings on the tube 573 that are associated with a specific tube channel. Thus, filler may be injected through the specific tube channel, via the openings on the tube 573, and into the inner chamber 571 for adjustment of the inner chamber 571's size. Likewise, the inner chamber 572 may be constructed by wrapping a membrane around another location on the tube 573 having one or more additional openings that are associated with a second specific tube channel. And filler may be injected through the second specific tube channel, via the additional openings, and into the inner chamber 572 for adjustment of the chamber 572's size. Thus, the inner chambers may be constructed by wrapping membranes around the tube 573 at different segments, wherein each segment may have one or more openings for filler or air inlet, and each segment may be configured to adjust a specific inner chamber.

In one embodiment, an implant 582 may be placed within another implant 581. Specifically, the implant 581 may be attached with a tube 585 for delivering filler 583 in or out of the implant 581. The implant 582 may be attached with a tube 586 for transporting filler 584 in or out of the implant 582. Thus, when filling the implant 581 and the implant 582 independently, the combination of the implants 581 and 582 may achieve an outer shape that cannot be easily achieve-able using a single implant. In one embodiment, the tube 586, which is connected to the inner implant 582, may be placed inside of the tube 585 that is connected to the outer implant 581. Alternatively, the tube 586 may connect to the inner implant 582 by passing through an opening on the outer implant 581. In other words, the "inner" implant 582 may be adjusted in similar fashions as an inner chamber of the implant 581.

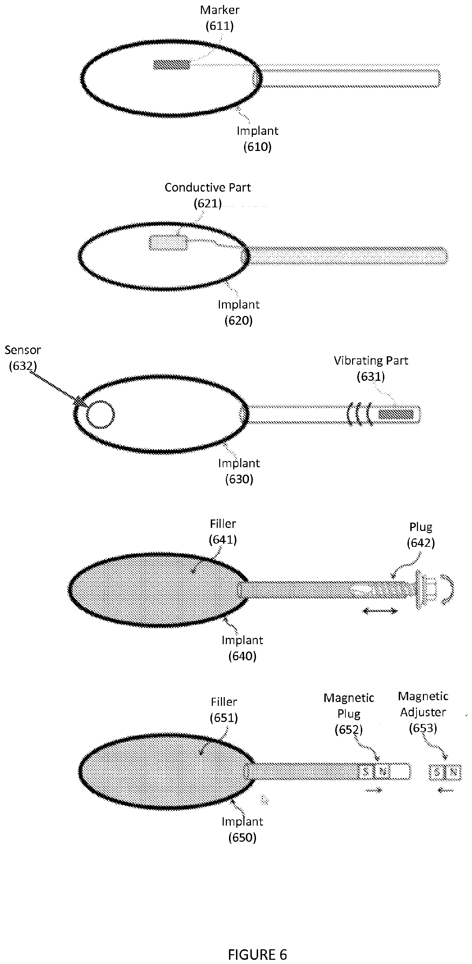

Figure 6 illustrates various components that may be associated with an implant for the treatment of glottic insufficiency, according to one or more embodiments of the present disclosure. In one embodiment, an implant 610 may have a marker 611 placed at a specific location either inside of the implant 160 or on the outer surface of the implant 160. The marker 611 may be made of radio-opaque materials. When the implant 610 is inflated and placed inside of a patient's body, a surgeon or a medical machinery may utilize an imaging device to search for the radio-opaque marker 611. The location of the radio-opaque marker 611 discovered by the imaging device may be used to indicate the implant 610's specific location inside the patient's body. The imaging device may utilize technologies such as video, optical coherence tomography, photo-acoustic, or ultrasound imaging to locate the radio-opaque marker 611, and to facilitate dissection, assess anatomical structure, and ensure good localization of the implant 610.

In one embodiment, an implant 620 may have a conductive part 621 on its surface. The conductive part 621 may be made of conductive material allowing electrical stimulation of the tissues surrounding the implant 620. During or after medical procedure, when the implant 610 is inflated and placed inside of a patient's body, a surgeon or a medical machinery may transmit electricity to the conductive part 621. The conductive part 621 may then perform electrical stimulation to facilitate rehabilitation or test the function of tissues surrounding the implant 620. In one embodiment, the frequency of the electrical stimulation may be adjusted to a specific value within a range from approximately 20Hz to approximately 20KHz.

In one embodiment, the electrical stimulation adjustment may be correlated to the anatomical location of the tissues and organs to be stimulated. In other words, for a specific anatomical location, the frequency and current of the electricity transmitted to the conductive part 621 may be adjusted or tuned to a specific value in order to generate satisfactory outcomes. For example, a location of the direct stimulation may be related to a target nerve, such as the recurrent laryngeal nerve, which plays an important role associated with the vocal closure. To direct stimulate the anterior branch of the inferior laryngeal nerve, the implant 620 may deliver more efficient effect when it is placed behind the arytenoid cartilage or the trans-arytenoid muscle area. For the direct stimulation over the posterior branch of the inferior laryngeal nerve, the implant 620 may be placed over the thyro-arytenoid muscle groups.

In one embodiment, an implant 630 may be connected with a vibrating part 631. The vibrating part 631 may be attached to the implant 630, either directly or via a tube that connects to the implant 630. The vibrating part 631 may be a piezo actuator, a syringe, or a fluid pump. Further, the frequency generated by the vibrating part 631 for the purposes of mechanical stimulation may be in a range between approximately 20Hz to approximately 20KHz. During operation, the vibrating part 631 may generate mechanical stimulations that can be transmitted to the tissues and organs surrounding the implant 630 and/or the tube.

In one embodiment, the implant 630 may be equipped with a sensor 632. The sensor 632 may be a pressure sensor. Once the implant 630 is installed near the vocal cord of a patient, the patient may be asked to cough during and after the procedure. The measurement generated by the pressure sensor 632 may be used as an indicator of the closure of the vocal cord, and thus guide the subsequent adjustment of the implant 630.

In one embodiment, the shape and the size of the implant 640 may be adjusted by changing the amount of filler 641 contained in the implant 640. For example, the implant 640 may have a plug 642 installed at one end of a tube that is connected to the implant 640. The plug 642 may have a screw mechanism, so that when the plug 642 is rotated in one direction (e.g. clockwise), it may push the amount of filler in the tube toward the implant 640. The additional amount of filler introduced into the implant 640 may cause the implant 640 to expand in volume. Likewise, a surgeon or a medical machinery may rotate the plug 642 in another direction (e.g., counter-clockwise), thereby freeing up space in the tube that was originally occupied by the plug 642. The freed up space in the tube may result in less filler 641 being in the implant 640, which may lead to the shrinking of the implant 640.

In one embodiment, the implant 650 may have a magnetic plug 652 installed at one end of a tube that is connected to the implant 650. The shape and the size of the implant 650 may be adjusted by pushing or pulling the magnetic plug 652 closer to or away from the implant 650. Depending on its magnet polarity, the magnetic adjuster 653 may either attract, or repel the magnetic plug 652, which may in turn push or pull the magnetic plug 652. The movement of the magnetic plug 652 in the tube may change the amount of filler 651 contained in the implant 650, which may lead to the expanding or shrinking of the implant 650. In one embodiment, the above adjustment of the filler in the implants 640 and 650 may be carried out intra-operatively or post-operatively.

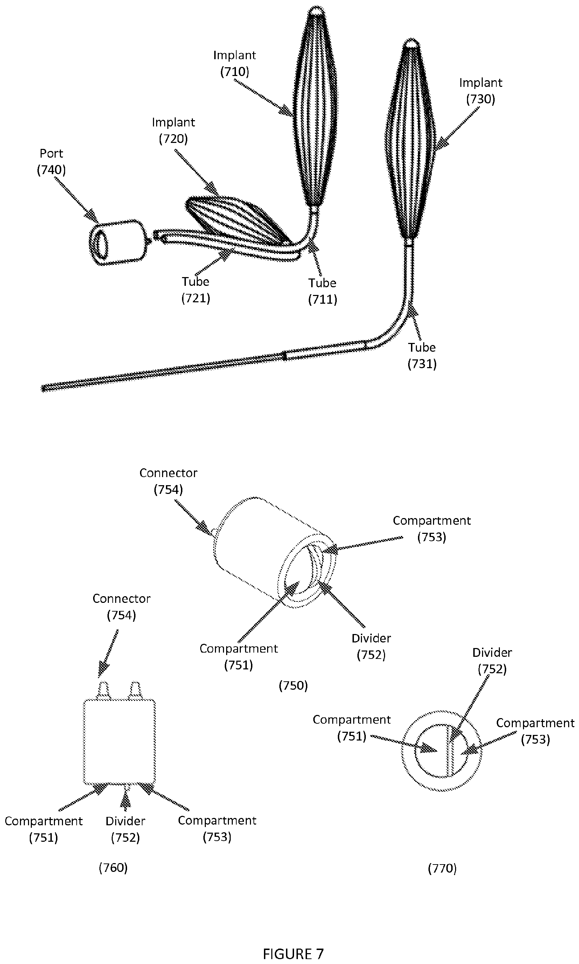

Figure 7 illustrates various diagrams of a port configured to connect to an implant for the treatment of glottic insufficiency, according to one or more embodiments of the present disclosure. In Figure 7, an implant 710 may be connected to a tube 711, an implant 720 may be connected to a tube 722, and an implant 730 may be connected to a tube 731. In one embodiment, the tube 711 and the tube 721 may have their one-ends connected to a port 740. The port 740 may be a device configured to maintain and deliver filler. Thus, the implant 710 or the implant 720 may be deemed in a semi-closed filler delivery system, as the port 740 may be used to deliver filler when "opened", and act as a valve when "closed." In other words, by using the port 740, filler may be added to or removed from the implant 710 or the implant 720 intra-operatively or postoperatively. In comparison, the tube 731 may be sealed off. Even when a mechanical or magnetic plug is installed in the tube 731 to adjust the volume of the implant 730, since no filler may be added into or removed from the implant 730 and the tube 731, the implant 730 may be deemed in a closed filler delivery system.

In one embodiment, the port 740 may be further illustrated by the drawings 750, 760, and 770. Specifically, the drawing 750 may show a 3D illustration of the port 740, the drawing 760 may show a side view of the port 740, and the drawing 770 may show a frontal view of the port 740. Further, the port 740 may contain one or more compartments 751 and 753 for adding/extracting fillers to/from corresponding implants or inner chambers of an implant. Multiple compartments may be separated by one or more dividers 752. In other words, each compartment in the port 740 may be separated from another compartment in the port 740 by one or more dividers 752. The port 740 may also contains one or more connectors 754 for connecting the compartments with the corresponding tubes. For example, the compartment 751 may be connected with the tube 711 via a first connector 754, and the compartment 753 may be connected with the tube 721 via a second connector 754.

In one embodiment, the port 740 may act as a delivery mechanism to add filler to, or extract filler from, the implants 710 and 720. Further, the port 740 may act as a valve to maintain the amount of filler in the implants 710 and 720 as well as in the tubes 711 and 721. In one embodiment, the port 740 may be constructed using materials such as septum. Thus, an injector may be used to add or extract filler via the septum. Once the injector completes the adding or extracting actions, the septum may automatically seal itself off, thereby maintaining the amount of filler in the semi-closed delivery system. For example, the injector may be inserted into the compartment 751, and inject an additional amount of filler into the septum in the compartment 751. The additional amount of filler may then flow out of the connector 754, into the tube 711, and into the implant 710.

In one embodiment, a portion of the divider 752 may extend beyond the cylinder body of the port 740. Further, the divider 752 may be positioned in such a way that the compartment 751 has a larger exposed surface size than the compartment 753. Thus, when the port 740 is secured underneath a patient's skin, a surgeon or a medical machinery may still be able to identify which of the two compartments 751 and 753 are connected with a specific implant, based on a touch and feel of the existence and the location of the portion of the divider 752 that extends beyond the body of the port 740. In other words, once a surgeon detects the divider 752's location in relative to the port 740, it may be able to differentiate the compartment 751 from the compartment 753. For example, the divider 752 may divide the frontal surface area of the port 740 into a large section associated with the compartment 751 and a small section associate with the compartment 752. During postoperative filler adjustment, the surgeon may detect that one section of the surface area is larger than another section of the surface area, based on the location of the divider 752 the surgeon senses. Since the surgeon may have prior knowledge that the large section of surface area is associated with the compartment 751, he may be able to direct the injector into the correct compartment for delivering filler to the implant that is connected with the compartment.

In one embodiment, the port 740 may contain a one-way valve which can be opened or closed for delivery of filler. For example, when the one-way valve is open, additional filler may be added into the implant. However, the one-way valve may prevent the removal of any filler, in order to maintain the amount of filler in the implant. Once the one-way valve is closed, then no filler may be added to the implant. In one embodiment, the one-way valve may be opened or closed mechanically through a direct mechanical force using a device such as a screw driver. Alternatively, the one-way valve may be an electric valve, which can be opened or closed electrically through an external electrical control device.

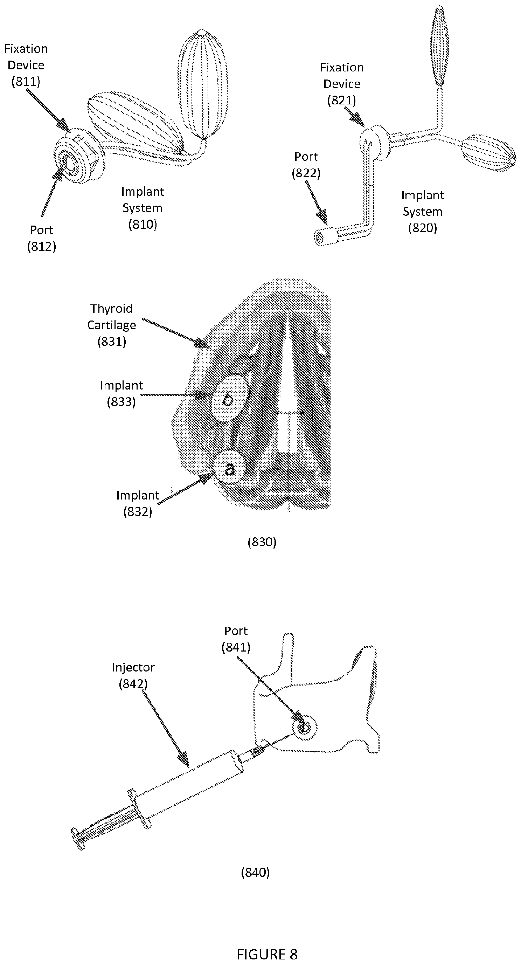

Figure 8 illustrates diagrams of an implant system configured to treat glottic insufficiency, according to one or more examples. In Figure 8, implant system 810 may include one or more implants positioned in a patient's paraglottic space, one or more tubes each of which having a first end coupled with one of the implants, a fixation device 811 fixated in a cartilage opening created in the patient's thyroid cartilage, and a port 812 to regulate an amount of filler in the one or more implants. Likewise, an implant system 820 may contain one or more implants, one or more tubes, a fixation device 821 fixated in a cartilage opening created in the patient's thyroid cartilage, and a port 822.

In one example, the fixation device 811 in the implant system 810 may have one channel having a cross-section diameter that is slightly larger than the cross-section diameter of the port 812. In this case, the port 812 may be placed into the fixation device in a way that no fluid is leaked out from the inside of the thyroid cartilage. Alternatively, the fixation device 821 in the implant system 820 may have multiple channels each of which is configured to fit a single tube or an instrument. In this case, the fixation device 821 may be installed in the cartilage opening, while the port 822 may be positioned outside of the thyroid cartilage and a distance away from the cartilage opening.

In one example, the implant system 810 or the implant system 820 may include two or more implants. To treat glottic insufficiency and dysphagia, one of the implants may be deployed in the paraglottic space to improve the glottis closure, while the other implant may be installed near the epiglottis to restore epiglottis bending and/or perform laryngeal elevation. For example, as shown in drawing 830, the implant 832 and the implant 833 may be placed in to two separate positions. To directly stimulate the anterior branch of the inferior laryngeal nerve, the implant 832 may deliver more efficient effect when it is placed behind the arytenoid cartilage or the trans-arytenoid muscle area. For the direct stimulation over the posterior branch of the inferior laryngeal nerve, the implant 833 may be placed over the thyro-arytenoid muscle groups. In one example, one or more implants may be delivered through or bypassing the thyroid cartilage, either through the junction between the thyroid ligament and the thyroid cartilage or through junction between the cricothyroid ligament and the thyroid cartilage 831.

In one example, there is a mechanism to wrap the remaining tube around, thus minimizes the space/distance between the implant and the port. For example, the fixation device has a dumbbell-shaped structure, and the remaining tube could be wrapped around the waist of the structure. Similar to that used in cord reels, the spring and other retracting mechanisms could be used to facilitate the retraction of the remaining tube.

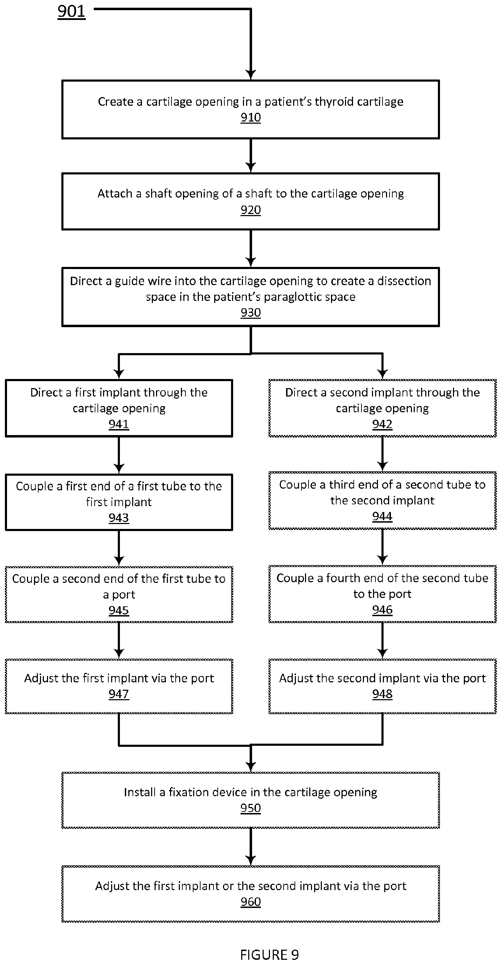

Figure 9 shows a flow diagram illustrating a process to treat a patient's glottic insufficiency, according to one or more examples. The processes 901 may set forth various functional blocks or actions that may be described as processing steps, medical procedures, medical operations, events, and/or acts, which may be performed by hardware, software, and/or a medical personal. Those skilled in the art in light of the present disclosure will recognize that numerous alternatives to the functional blocks shown in Figure 9 may be practiced in various implementations.

One skilled in the art will appreciate that, for this and other processes and methods disclosed herein, the functions performed in the processes and methods may be implemented in differing order. Furthermore, the outlined steps and operations are only provided as examples, and some of the steps and operations may be optional, combined into fewer steps and operations, or expanded into additional steps and operations without detracting from the essence of the disclosed examples. Moreover, one or more of the outlined steps and operations may be performed in parallel.

At block 910, a surgeon or a medical machinery may utilize a surgical device to create a cartilage opening in a patient's thyroid cartilage. The surgical device may include a surgical tool configured to remove at least a portion of the cartilage from the thyroid cartilage and create the cartilage opening. The surgical device may further include a shaft coupled with the surgical tool. The shaft may have a shaft opening. At block 920, the surgeon or the medical machinery may attach the shaft opening of the shaft to the cartilage opening. At block 930, the surgeon or the medical machinery may direct a guide wire through the shaft via the shaft opening into the cartilage opening to create a dissection space in the patient's paraglottic space.

In one example, the implant system may contain a first implant and a second implant, and the process 901 may proceed to block 941 to install the first implant, or to block 942 to install the second implant.

At block 941, the surgeon or the medical machinery may direct a first implant through the cartilage opening via the shaft. In one example, the surgeon or the medical machinery may insert a guide wire into the first implant, and direct the first implant into a desired location in the patient's paraglottic space by controlling the handle of the guide wire. To treat glottic insufficiency, the first implant may be placed in the larynx to hold one of the patient's vocal folds to a particular displacement position.

At block 943, once the first implant is installed to a desired location in the paraglottic space, the surgeon or the medical machinery may couple a first tube to the first implant. Specifically, the first tube may have a first end and a second end, and the first implant may be coupled to the first end of the first tube. Alternatively, the implant and the first tube may be integrated as a single piece. In this case, the first tube may have its first end being a part of the implant, and its second end configured to be coupled with additional components. At block 945, the surgeon or the medical machinery may couple the second end of the first tube to a port of the implant system. In one example, the port may contain a first compartment and a second compartment, and the first tube may be coupled to the first compartment of the port.

At block 947, the surgeon or the medical machinery may perform an intraoperative adjustment of the first implant by adding/retracting fillers to/from the first implant via the port and the first tube. Specifically, the port may contain septum, and the surgeon or the medical machinery may utilize an injector to inject filler into, or draw filler from, the first implant via the port's first compartment. The adjustment of the first implant may push the patient's paralyzed vocal fold to a "medialized" position.

At block 942, the surgeon or the medical machinery may direct a second implant through the cartilage opening via the shaft. In one example, the surgeon or the medical machinery may insert the guide wire into the second implant, and direct the second implant into a desired location in the patient's paraglottic space by controlling the handle of the guide wire. To assist the patient's epiglottis and larynx, the second implant may be placed at a particular (which may be predetermined) position that can help the epiglottis bending movements or laryngeal elevation.

At block 944, once the second implant is installed to a desired location in the paraglottic space, the surgeon or the medical machinery may couple a second tube to the second implant. Specifically, the second tube may have a third end and a fourth end, and the second implant may be coupled to the third end of the second tube. Alternatively, the second implant and the second tube may be integrated as a single piece. Similar to the first implant, the second tube's third end may be a part of the implant, leaving the fourth end to couple with additional components. At block 946, the surgeon or the medical machinery may couple the fourth end of the second tube to the port of the implant system.

In one example, the first tube may be connected to the first compartment, and the second tube may be connected to the second compartment, of the port. In another example, when the first tube and the second tube are connected with two inner compartments of a single implant, the port's first compartment and the second compartment may be used to individually adjust these two inner compartments of a single implant.

At block 948, the surgeon or the medical machinery may perform an intraoperative adjustment of second implant by adding/retracting fillers to/from the second implant via the port and the second tube. Specifically, the surgeon or the medical machinery may utilize the injector to inject filler into, or draw filler from, the first implant via the port's second compartment.

At block 950, the surgeon or the medical machinery may install a fixation device, which is a part of the implant system, to the cartilage opening. Once installed, the fixation device may have at least a channel allowing the first tube or the second tube to pass through the cartilage opening. In one example, the port may be installed in the fixation device's channel. In another example, the fixation device may have one or more channels to hold one or more tubes, and the port may be fixated at a location away from the cartilage opening.

At block 960, the surgeon or the medical machinery may perform a postoperative adjustment of the first implant or the second implant by adding/retracting filler to/from the first implant via the first compartment of the port, or adding/retracting filler to/from the second implant via the second compartment of the port.

Thus, apparatuses and methods for treating glottic insufficiency have been disclosed. The various examples described herein may employ a device or an apparatus for performing these methods. The apparatus may be specially constructed for specific required purposes, or it may be a general purpose computer selectively activated or configured by a computer program stored in the computer. In particular, various general purpose machines may be used with computer programs written in accordance with the teachings herein, or it may be more convenient to construct a more specialized apparatus to perform the required operations. The various examples described herein may be practiced with other computer system configurations including hand-held devices, microprocessor systems, microprocessor-based or programmable consumer electronics, minicomputers, mainframe computers, and the like.

Although one or more embodiments of the present disclosure have been described in some detail for clarity of understanding, it will be apparent that certain changes and modifications may be made within the scope of the claims. Accordingly, the described embodiments are to be considered as illustrative and not restrictive, and the scope of the claims is not to be limited to details given herein, but may be modified within the scope and equivalents of the claims. In the claims, elements and/or steps do not imply any particular order of operation, unless explicitly stated in the claims.

Plural instances may be provided for components, operations or structures described herein as a single instance. Finally, boundaries between various components, operations and data stores are somewhat arbitrary, and particular operations are illustrated in the context of specific illustrative configurations. Other allocations of functionality are envisioned and may fall within the scope of the disclosure(s). In general, structures and functionality presented as separate components in exemplary configurations may be implemented as a combined structure or component. Similarly, structures and functionality presented as a single component may be implemented as separate components. These and other variations, modifications, additions, and improvements may fall within the scope of the appended claims(s).

## Claims

1. An implant system configured to treat a patient's glottic insufficiency, comprising:
a first implant (561; 571; 581; 710) configured to be positioned in the patient's paraglottic space, wherein the first implant (561; 571; 581; 710) is adjustable in volume by changing amount of filler in the first implant (561; 571; 581; 710);
a fixation device (430) configured to be fixated on or through a cartilage opening in the patient's thyroid cartilage, wherein the fixation device (430) contains a first channel;
a first tube (563; 573; 585; 711) having a first end coupled with the first implant (561; 571; 581; 710), wherein the first tube (563; 573; 585; 711) delivers the filler to the first implant (561; 571; 581; 710); and
a port (740) coupled with a second end of the first tube (563; 573; 585; 711) to regulate the amount of filler in the first implant (561; 571; 581; 710);
**characterised by** further comprising:
a second implant (564; 572; 582; 720) configured to be positioned in the patient's paraglottic space; and
a second tube (566; 573; 586; 721) having a third end coupled with the second implant (564; 572; 582; 720), having a fourth end coupled with the port (740), and configured to pass through the cartilage opening in the thyroid cartilage via the fixation device (430);
wherein:
the port (740) contains a first compartment (751) connected to the first tube (563; 573; 585; 711) and a second compartment (753) connected to the second tube (566; 573; 586; 721), and a configurable amount of the filler can be added to the first implant (561; 571; 581; 710) via the first compartment (751) to adjust volume of the first implant (561; 571; 581; 710), and to the second implant (564; 572; 582; 720) via the second compartment (753) to adjust volume of the second implant (564; 572; 582; 720).

2. The implant system of claim 1, wherein the first tube (563; 573; 585; 711) is configured to pass through the first channel of the fixation device (430) to deliver the filler to the first implant (561; 571; 581; 710).

3. The implant system of claim 1, wherein the first tube (563; 573; 585; 711) is configured to pass through the cartilage opening along with the fixation device (430).

4. The implant system of claim 1, wherein the port (740) contains septum, and the first implant (561; 571; 581; 710) is expandable or retractable in volume by adding or retracting via the port's septum a configurable amount of the filler to or from the first implant (561; 571; 581; 710).

5. The implant system of claim 1, wherein the first implant (561; 571; 581; 710) is configured to be placed in the larynx to hold the patient's vocal fold in a particular displacement position.

6. The implant system of claim 1, wherein the first implant (561; 571; 581; 710) is configured to be placed in the larynx to assist the patient's epiglottis and larynx during swallowing.

7. The implant system of claim 1, further comprising a marker (611) coupled with the first implant (561; 571; 581; 710) to disclose a position of the first implant (561; 571; 581; 710) in the patient's paraglottic space.

8. The implant system of claim 1, further comprising a conductive part (621) coupled with the first implant to electrically stimulate the patient's tissues surrounding the first implant (561; 571; 581; 710).

9. The implant system of claim 1, further comprising a vibrating part (631) coupled with the first implant (561; 571; 581; 710) to mechanically stimulate the patient's tissues surrounding the first implant (561; 571; 581; 710).

10. The implant system of claim 1, further comprising a pressure sensor (632) coupled with the first implant (561; 571; 581; 710) to measure a closure of the patient's vocal cord.

11. The implant system of claim 1, wherein the fixation device contains a first fixture (411) configured to contact one side of the thyroid cartilage and a second fixture (412) contact another side of the thyroid cartilage, and the first fixture (411) and the second fixture (412) are operable to clamp the fixation device (430) to the thyroid cartilage through the cartilage opening in the thyroid cartilage.

12. The implant system of claim 1, wherein the fixation device's (430) first channel has an outer surface configured to contact the cartilage opening in the thyroid cartilage and providing friction to maintain the fixation device (430) in the cartilage opening.

13. The implant system of claim 1, further comprising at least one of:
a screw-shaped plug (642) coupled with the first tube (563; 573; 585; 711), wherein rotation of the screw-shaped plug (642) adjusts the filler in the first implant (561; 571; 581; 710); and
a magnetic plug (652) coupled with the first tube (563; 573; 585; 711), wherein manipulation of the magnetic plug (652) with a magnet adjuster adjusts the filler in the first implant (561; 571; 581; 710).

14. The implant system of claim 1, wherein the port (740):
contains a valve, and closure of the valve stops the first implant (561; 571; 581; 710) from being adjusted; and/or
is placed within the fixation device's (430) first channel.

15. The implant system of claim 1, wherein:
the second implant (564; 572; 582; 720) is placed inside of the first implant (561; 571; 581; 710), and the second tube (566; 573; 586; 721) is placed inside of the first tube (563; 573; 585; 711); and/or
the fixation device (430) contains a second channel, and the second tube (566; 573; 586; 721) is configured to pass through the cartilage opening in the thyroid cartilage via the second channel.

## Patentansprüche

1. Implantatsystem, dazu konfiguriert, Stimmritzenfehler eines Patienten zu behandeln, umfassend:
ein erstes Implantat (561; 571; 581; 710), dazu konfiguriert, im paraglottischen Raum des Patienten positioniert zu werden, wobei das Volumen des ersten Implantats (561; 571; 581; 710) durch Verändern der Menge an Füllstoff im ersten Implantat (561; 571; 581; 710) einstellbar ist;
eine Befestigungsvorrichtung (430), dazu konfiguriert, auf oder durch eine Knorpelöffnung im Schildknorpel des Patienten befestigt zu werden, wobei die Befestigungsvorrichtung (430) einen ersten Kanal enthält;
ein erstes Rohr (563; 573; 585; 711) mit einem mit dem ersten Implantat (561; 571; 581; 710) gekoppelten ersten Ende, wobei das erste Rohr (563; 573; 585; 711) den Füllstoff dem ersten Implantat (561; 571; 581; 710) zuführt; und
eine Öffnung (740), die mit einem zweiten Ende des ersten Rohres (563; 573; 585; 711) gekoppelt ist, um die Menge an Füllstoff im ersten Implantat (561; 571; 581; 710) zu regulieren;
**gekennzeichnet dadurch, dass** es ferner Folgendes umfasst:
ein zweites Implantat (564; 572; 582; 720), dazu konfiguriert, im paraglottischen Raum des Patienten positioniert zu werden; und
ein zweites Rohr (566; 573; 586; 721) mit einem mit dem zweiten Implantat (564; 572; 582; 720) gekoppelten dritten Ende, mit einem mit der Öffnung (740) gekoppelten vierten Ende und dazu konfiguriert durch die Knorpelöffnung im Schildknorpel über die Befestigungsvorrichtung (430) hindurchzutreten;
wobei:
die Öffnung (740) eine mit dem ersten Rohr (563; 573; 585; 711) verbundene erste Kammer (751) und eine mit dem zweiten Rohr (566; 573; 586; 721) verbundene zweite Kammer (753) enthält, und eine konfigurierbare Menge des Füllstoffs dem ersten Implantat (561; 571; 581; 710) über die erste Kammer (751) zum Einstellen des Volumens des ersten Implantats (561; 571; 581; 710) und dem zweiten Implantat (564; 572; 582; 720) über die zweite Kammer (753) zum Einstellen des Volumens des zweiten Implantats (564; 572; 582; 720) zugegeben werden kann.

2. Implantatsystem nach Anspruch 1, wobei das erste Rohr (563; 573; 585; 711) dazu konfiguriert ist, durch den ersten Kanal der Befestigungsvorrichtung (430) hindurchzutreten, um den Füllstoff dem ersten Implantat zuzuführen (561; 571; 581; 710).

3. Implantatsystem nach Anspruch 1, wobei das erste Rohr (563; 573; 585; 711) dazu konfiguriert ist, durch die Knorpelöffnung zusammen mit der Befestigungsvorrichtung (430) hindurchzutreten.

4. Implantatsystem nach Anspruch 1, wobei die Öffnung (740) ein Septum enthält und das Volumen des ersten Implantats (561; 571; 581; 710) vergrößerbar oder verkleinerbar ist, indem über das Septum der Öffnung eine konfigurierbare Menge des Füllstoffs dem ersten Implantat (561; 571; 581; 710) zugegeben oder diesem entnommen wird.

5. Implantatsystem nach Anspruch 1, wobei das erste Implantat (561; 571; 581; 710) dazu konfiguriert ist, im Kehlkopf angeordnet zu werden, um die Stimmfalte des Patienten in einer bestimmten Verschiebungsposition zu halten.

6. Implantatsystem nach Anspruch 1, wobei das erste Implantat (561; 571; 581; 710) dazu konfiguriert ist, im Kehlkopf angeordnet zu werden, um die Epiglottis und den Kehlkopf des Patienten beim Schlucken zu unterstützen.

7. Implantatsystem nach Anspruch 1, ferner umfassend eine mit dem ersten Implantat (561; 571; 581; 710) gekoppelte Markierung (611), um eine Position des ersten Implantats (561; 571; 581; 710) im paraglottischen Raum des Patienten zu offenbaren.

8. Implantatsystem nach Anspruch 1, ferner umfassend ein mit dem ersten Implantat gekoppeltes leitfähiges Teil (621), um die das erste Implantat (561; 571; 581; 710) umgebenden Gewebe des Patienten elektrisch zu stimulieren.

9. Implantatsystem nach Anspruch 1, ferner umfassend ein mit dem ersten Implantat (561; 571; 581; 710) gekoppeltes vibrierendes Teil (631), um die das erste Implantat (561; 571; 581; 710) umgebenden Gewebe des Patienten mechanisch zu stimulieren.

10. Implantatsystem nach Anspruch 1, ferner umfassend einen mit dem ersten Implantat (561; 571; 581; 710) gekoppelten Drucksensor (632), um einen Verschluss des Stimmbandes des Patienten zu messen.

11. Implantatsystem nach Anspruch 1, wobei die Befestigungsvorrichtung eine erste Halterung (411), dazu konfiguriert, eine Seite des Schildknorpels zu berühren, und eine zweite Halterung (412) zum Berühren einer anderen Seite des Schildknorpels enthält und die erste Halterung (411) und die zweite Halterung (412) betriebsfähig sind, die Befestigungsvorrichtung (430) durch die Knorpelöffnung im Schildknorpel am Schilddrüsenknorpel festzuklammern.

12. Implantatsystem nach Anspruch 1, wobei der erste Kanal der Befestigungsvorrichtung (430) eine Außenfläche aufweist, dazu konfiguriert, die Knorpelöffnung im Schildknorpel zu berühren und Reibung bereitzustellen, um die Befestigungsvorrichtung (430) in der Knorpelöffnung zu halten.

13. Implantatsystem nach Anspruch 1, ferner umfassend mindestens eins der Folgenden:
einen mit dem ersten Rohr (563; 573; 585; 711) gekoppelten schraubenförmigen Stopfen (642), wobei eine Drehung des schraubenförmigen Stopfens (642) den Füllstoff im ersten Implantat (561; 571; 581; 710) einstellt; und
einen mit dem ersten Rohr (563; 573; 585; 711) gekoppelten Magnetstopfen (652), wobei eine Betätigung des Magnetstopfens (652) mit einer Magneteinstellvorrichtung den Füllstoff im ersten Implantat (561; 571; 581; 710) einstellt.

14. Implantatsystem nach Anspruch 1, wobei die Öffnung (740):
ein Ventil enthält und ein Schließen des Ventils die Einstellung des ersten Implantats (561; 571; 581; 710) stoppt; und/oder
im ersten Kanal der Befestigungsvorrichtung (430) angeordnet ist.

15. Implantatsystem nach Anspruch 1, wobei:
das zweite Implantat (564; 572; 582; 720) innerhalb des ersten Implantats (561; 571; 581; 710) angeordnet ist und das zweite Rohr (566; 573; 586; 721) innerhalb des ersten Rohres (563; 573; 585; 711) angeordnet ist; und/oder
die Befestigungsvorrichtung (430) einen zweiten Kanal enthält und das zweite Rohr (566; 573; 586; 721) dazu konfiguriert ist, durch die Knorpelöffnung im Schildknorpel über den zweiten Kanal hindurchzutreten.

## Revendications

1. Système d'implant conçu pour traiter une insuffisance glottique chez un patient, comprenant :
un premier implant (561 ; 571 ; 581 ; 710) conçu pour être positionné dans l'espace paraglottique du patient, dans lequel le premier implant (561 ; 571 ; 581 ; 710) est ajustable en volume en modifiant la quantité de charge dans le premier implant (561 ; 571 ; 581 ; 710) ;
un dispositif de fixation (430) conçu pour être fixé sur ou à travers une ouverture du cartilage dans le cartilage thyroïdien du patient, dans lequel le dispositif de fixation (430) comprend un premier canal ;
un premier tube (563 ; 573 ; 585 ; 711) ayant une première extrémité couplée au premier implant (561 ; 571 ; 581 ; 710), dans lequel le premier tube (563 ; 573 ; 585 ; 711) délivre la charge au premier implant (561 ; 571 ; 581 ; 710) ; et
un orifice (740) couplé à une deuxième extrémité du premier tube (563 ; 573 ; 585 ; 711) pour réguler la quantité de charge dans le premier implant (561 ; 571 ; 581 ; 710) ;
caractérisé en comprenant également :
un second implant (564 ; 572 ; 582 ; 720) conçu pour être positionné dans l'espace paraglottique du patient ; et
un second tube (566 ; 573 ; 586 ; 721) ayant une troisième extrémité couplée au second implant (564 ; 572 ; 582 ; 720), ayant une quatrième extrémité couplée à l'orifice (740), et conçu pour passer à travers l'ouverture du cartilage dans le cartilage thyroïdien à travers le dispositif de fixation (430) ;
dans lequel :
l'orifice (740) contient un premier compartiment (751) relié au premier tube (563 ; 573 ; 585 ; 711) et un second compartiment (753) relié au second tube (566 ; 573 ; 586 ; 721), et une quantité configurable de la charge peut être ajoutée au premier implant (561 ; 571 ; 581 ; 710) à travers le premier compartiment (751) pour ajuster le volume du premier implant (561 ; 571 ; 581 ; 710), et au second implant (564 ; 572 ; 582 ; 720) à travers le second compartiment (753) pour ajuster le volume du second implant (564 ; 572 ; 582 ; 720).

2. Système d'implant de la revendication 1, dans lequel le premier tube (563 ; 573 ; 585 ; 711) est conçu pour passer à travers le premier canal du dispositif de fixation (430) pour délivrer la charge au premier implant (561 ; 571 ; 581 ; 710).

3. Système d'implant de la revendication 1, dans lequel le premier tube (563 ; 573 ; 585 ; 711) est conçu pour passer à travers l'ouverture du cartilage accompagné du dispositif de fixation (430).

4. Système d'implant de la revendication 1, dans lequel l'orifice (740) contient une cloison, et le premier implant (561 ; 571 ; 581 ; 710) peut être déployé ou rétracté en volume en ajoutant ou en enlevant à travers la cloison de l'orifice une quantité configurable de la charge vers ou à partir du premier implant (561 ; 571 ; 581 ; 710).

5. Système d'implant de la revendication 1, dans lequel le premier implant (561 ; 571 ; 581 ; 710) est conçu pour être placé dans le larynx afin de maintenir les replis vocaux du patient dans une position de déplacement donnée.

6. Système d'implant de la revendication 1, dans lequel le premier implant (561 ; 571 ; 581 ; 710) est conçu pour être placé dans le larynx afin d'aider l'épiglotte et le larynx du patient pendant la déglutition.

7. Système d'implant de la revendication 1, comprenant également un marqueur (611) couplé au premier implant (561 ; 571 ; 581 ; 710) pour révéler une position du premier implant (561 ; 571 ; 581 ; 710) dans l'espace paraglottique du patient.

8. Système d'implant de la revendication 1, comprenant également une partie conductrice (621) couplée au premier implant pour stimuler électriquement les tissus du patient entourant le premier implant (561 ; 571 ; 581 ; 710).

9. Système d'implant de la revendication 1, comprenant également une partie vibrante (631) couplée au premier implant (561 ; 571 ; 581 ; 710) pour stimuler mécaniquement les tissus du patient entourant le premier implant (561 ; 571 ; 581 ; 710).

10. Système d'implant de la revendication 1, comprenant également un capteur de pression (632) couplé au premier implant (561 ; 571 ; 581 ; 710) pour mesurer une fermeture de la corde vocale du patient.

11. Système d'implant de la revendication 1, dans lequel le dispositif de fixation contient une première fixation (411) conçue pour entrer en contact avec un côté du cartilage thyroïdien et une seconde fixation (412) pour entrer en contact avec un autre côté du cartilage thyroïdien, et la première fixation (411) et la seconde fixation (412) fonctionnent pour fixer le dispositif de fixation (430) au cartilage thyroïdien à travers l'ouverture du cartilage dans le cartilage thyroïdien.

12. Système d'implant de la revendication 1, dans lequel le premier canal du dispositif de fixation (430) comporte une surface externe conçue pour entrer en contact avec l'ouverture du cartilage dans le cartilage thyroïdien et pour fournir une friction afin de maintenir le dispositif de fixation (430) dans l'ouverture de cartilage.

13. Système d'implant de la revendication 1, comprenant également au moins l'un :
d'un bouchon en forme de vis (642) couplé au premier tube (563 ; 573 ; 585 ; 711), dans lequel la rotation du bouchon en forme de vis (642) ajuste la charge dans le premier implant (561 ; 571 ; 581 ; 710) ; et
un bouchon magnétique (652) couplé au premier tube (563 ; 573 ; 585 ; 711), dans lequel la manipulation du bouchon magnétique (652) avec un aimant ajusteur ajuste la charge dans le premier implant (561 ; 571 ; 581 ; 710).

14. Système d'implant de la revendication 1, dans lequel l'orifice (740) :
contient une valve, et la fermeture de la valve arrête l'ajustement du premier implant (561 ; 571 ; 581 ; 710) ; et/ou
est placé à l'intérieur du premier canal du dispositif de fixation (430).

15. Système d'implant de la revendication 1, dans lequel :
le second implant (564 ; 572 ; 582 ; 720) est placé à l'intérieur du premier implant (561 ; 571 ; 581 ; 710), et le second tube (566 ; 573 ; 586 ; 721) est placé à l'intérieur du premier tube (563 ; 573 ; 585 ; 711) ; et/ou
le dispositif de fixation (430) comporte un second canal, et le second tube (566 ; 573 ; 586 ; 721) est conçu pour passer à travers l'ouverture du cartilage dans le cartilage thyroïdien à travers le second canal.
